# EUROPÄISCHE PATENTANMELDUNG

(11) **EP 0 820 756 A1**
(43) Veröffentlichungstag der Anmeldung: **28.01.1998**
(21) Anmeldenummer: 97112313.8
(22) Anmeldetag: 17.07.1997
(51) Int. Cl.: A61K 7/00

(54) **Zubereitungen mit vom Betrachtungswinkel abhängiger Farbigkeit**

(30) Priorität: 23.07.1996 DE 19629761
(71) Anmelder: Wacker-Chemie GmbH, 81737 München (DE)
(72) Erfinder: Müller-Rees, Christoph, Dr., 82049 Pullach (DE); Huber, Annemarie, 84533 Haiming (DE)
(74) Vertreter: Potten, Holger

(57) **Zusammenfassung**

Die Erfindung betrifft kosmetische oder pharmazeutische Zubereitungen enthaltend Pigmente mit vom Betrachtungswinkel abhängiger Farbigkeit und die für kosmetischen oder pharmazeutischen Zubereitungen üblichen Zusatzstoffe, dadurch gekennzeichnet, daß die Pigmente mindestens eine orientierte vernetzte Substanz mit flüssigkristalliner Struktur mit chiraler Phase enthalten, von plättchenförmiger Gestalt sind und eine Dicke von 1 - 20 µm haben.

## Beschreibung

Die Erfindung betrifft Zubereitungen mit vom Betrachtungswinkel abhängiger Farbigkeit.

Kosmetische und/oder pharmazeutische Zubereitungen, beispielsweise zur Behandlung von Haaren, Nägeln und Haut enthaltend Pigmente sind bereits vielfach bekannt. Häufig besteht ein Bedarf an Effektpigmenten, die den Zubereitungen einen metallischen Farbglanz vermitteln und die physiologisch unbedenkliche, in der Kosmetik zugelassene Farbstoffe enthalten.

Es ist bekannt, dafür plättchenförmige Pigmente einzusetzen, wie z. B. die in den Patenten US-A-3711308 (entspricht DE-A 19 59 998), US-A-3874890 (entspricht DE-A-22 44 298) und US-A-3926659 (entspricht DE-A-23 13 331) beschriebenen Metalloxid/Glimmer-Pigmente. Dabei handelt es sich um dünne Glimmerplättchen, die mit Eisenoxid und ggf. anderen Metalloxiden beschichtet sind. Diese Glimmerpigmente weisen auf der Glimmeroberfläche zahllose kleinste Metalloxidkristalle nebeneinander abgeschieden auf und besitzen daher eine, mikroskopisch gesehen, relativ rauhe Oberfläche.

Aus US-5362315 (entspricht EP-A-601483) und EP-A-686674 sind Pigmente mit vom Betrachtungswinkel abhängiger Farbigkeit bekannt. In EP-A-686674 ist ferner erwähnt, daß sich diese Pigmente neben zahlreichen anderen Anwendungen auch zur Verwendung im Kosmetikbereich eignen, weitere Angaben dazu finden sich jedoch nicht.

Aufgabe vorliegender Erfindung war es, kosmetische oder pharmazeutische Zubereitungen zur Verfügung zu stellen, die einen hohen Glanz sowie vom Betrachtungswinkel abhängige Farbeffekte zeigen und die ein sehr gutes Hautfeeling besitzen.

Die Erfindung betrifft kosmetische oder pharmazeutische Zubereitungen enthaltend Pigmente mit vom Betrachtungswinkel abhängiger Farbigkeit und die für kosmetischen oder pharmazeutischen Zubereitungen üblichen Zusatzstoffe, dadurch gekennzeichnet, daß die Pigmente mindestens eine orientierte vernetzte Substanz mit flüssigkristalliner Struktur mit chiralen Phase enthalten, von plättchenförmiger Gestalt sind und eine Dicke von 1 - 20 µm haben.

Vorzugweise haben die Pigmente eine Dicke von 4 - 8 µm, besonders bevorzugt 4 bis 6 µm.

Vorzugweise haben die Pigmente einen Korndurchmesser von 5 - 500 µm, besonders bevorzugt 10 bis 100 µm.

Die erfindungsgemäßen Zubereitungen sind aufgrund des Verzichts auf schwermetallhaltige Pigmente umweltfreundlich.

Die in den erfindungsgemäßen Zubereitungen vorhandenen Pigmente werden beispielsweise wie in US-5362315 (entspricht EP-A-601483) oder in EP-A-686674 beschrieben hergestellt, mit dem Unterschied, daß bei der Zerkleinerung der Materialien zu den Pigmenten die obengenannten Kriterien bezüglich Form, Dicke und ggf. Korndurchmesser eingehalten werden oder die entsprechenden Teilchen nach dem Zerkleinern mittels bekannter Verfahren wie z.B. dem Klassieren ausgewählt werden.

Die Pigmente liegen in den erfindungsgemäßen Zusammensetzungen vorzugsweise in Mengen von 0,05 - 40 % (w/w), bevorzugt 0,1 - 20 % (w/w), besonders bevorzugt 1 - 5 % (w/w) vor.

Vorzugsweise bestehen die erfindungsgemäß eingesetzten Pigmente aus Polyorganosiloxanen.

Überraschend zeigte sich, daß die erfindungsgemäßen Zubereitungen einen hohen Glanz, vielfältige, besondere vom Betrachtungswinkel abhängige tiefenwirksame Farbeffekte aufweisen und ferner völlig unerwartet ein sehr gutes Hautfeeling sowie ein ausgeprägtes Absorptionsvermögen im UV-Bereich besitzen. Vorteilhafterweise sind die erfindungsgemäßen Formulierungen einfacher herzustellen als bekannte kosmetische oder pharmazeutische Zubereitungen, da sich die Pigmente mit vom Betrachtungswinkel abhängiger Farbigkeit leichter als die üblichen Pigmente in die jeweiligen Formulierungen einarbeiten lassen.

Die erfindungsgemäßen Zubereitungen können mittels bekannter und üblicher Verfahren hergestellt werden. Das Einarbeiten der genannten Pigmente kann in üblicher Art und Weise erfolgen.

Bei den erfindungsgemäßen Zusammensetzungen kann es sich beispielsweise um Präparate zur Behandlung von Haut, Nägeln und Haaren handeln. Beispiele hierfür sind Emulsionen, wie Reinigungsemulsionen, flüssige Nährcremes, Körperlotionen, Sonnenschutzmittel und Bademilch, Cremes, wie etwa feste Cremes nach Art einer Nachtcreme, Hautnährcreme, Sonnenschutzcreme und dergleichen, sowie Stifte, wie Deodorantstift, Lippenstifte und Augenschminkstifte. Die verwendeten Stoffe sind dem Fachmann bekannt oder können aus Standardwerken, entnommen werden.

Beispiele für Zusatzstoffe, die in den Zusammensetzungen enthalten sein können, sind tierische, mineralische, pflanzliche oder synthetische Öle, Wachse oder Harze, Netzmittel, Fettalkohole, Emulgatoren, Sonnenfilter, organische Lösungsmittel, Verdickungsmittel, Farbstoffe, Pigmente, pH-Regler, Reduk tionsmittel, Elektrolyte, Trübungszusätze, Konservierungsmittel, Antioxidantien, Permuttiermittel, Parfums, antiseborrhoische Mittel und Wirkstoffe, die der Behandlung, der Pflege und dem Schutz der Haut oder der Haare dienen können, sowie Wasser.

Art und Menge der Zusatzstoffe hängen vom jeweiligen Einsatzgebiet ab und sind im Bereich der Kosmetik bekannt.

Die erfindungsgemäßen Zubereitungen enthalten als färbende Bestandteile in jedem Fall zumindest eines der oben genannten plättchenförmigen Pigmente mit vom Betrachtungswinkel abhängiger Farbigkeit. Daneben können jedoch weitere Pigmente Zugemischt werden, wobei sowohl organische als auch anorganische Absorptionsfarbpigmente, Silberglanzpigmente, wie z. B. Wismutoxichlorid, Fischsilber oder Titandioxid-beschichteter Glimmer oder Interferenzfarbglanzpigmente auf Basis von mit Metalloxiden, insbesondere TiO₂, beschichteten Glimmerplättchen eingesetzt werden.

Insbesondere durch die Kombination mit Interferenzfarbpigmenten oder Metallglanzpigmenten werden sehr ansprechende Farbeffekte erzielt, so daß vorzugsweise solche Pigmente ebenfalls in der erfindungsgemäßen Zubereitung vorhanden sind.

Die vorliegende Erfindung stellt somit sehr vorteilhafte, neue kosmetische Zubereitungen mit sehr schönen Farbeffekten und einem sehr angenehmen Hautfeeling zur Verfügung.

In den nachstehenden Beispielen beziehen sich alle Angaben von Teilen und Prozentsätzen, falls nicht anders angegeben, auf das Gewicht. Des weiteren beziehen sich alle Viskositätsangaben auf eine Temperatur von 25 °C. Sofern nicht anders angegeben, wurden die nachstehenden Beispiele bei einem Druck der umgebenden Atmosphäre, also etwa 1000 hPa, und bei Raumtemperatur, also bei etwa 20 °C, bzw. bei einer Temperatur, die sich beim Zusammengeben der Reaktanden bei Raumtemperatur ohne zusätzliche Heizung oder Kühlung einstellt, durchgeführt.

Die Herstellung der erfindungsgemäßen Formulierungen erfolgte nach in der Kosmetikindustrie üblichen Methoden und mit gängigen Rohstoffen.

Die Beurteilung der Formulierungsmuster erfolgte durch zwei oder drei Personen, wobei Aussehen und Anwendung subjektiv beurteilt wurden.

### Beispiel 1: Gesichtspuder

Es wurden 70,00 % Talkum, 6,00 % Magnesiumstearat, 3,00 % Acrylsäurepolymerisat, erhältlich bei der Fa. Goodrich unter der Bezeichnung Carbopol 1342 vermischt und portionsweise 12,00 % eines leichtflüchtigen cyclischen Polydimethylsiloxans, käuflich erhältlich bei der Fa. Wacker-Chemie, München, unter der Bezeichnung Wacker-Belsil CM 040, und 2,00 % Proteinhydrolysat käuflich erhältlich bei der Fa. Grünau unter der Bezeichnung Nutrilan L zugemischt.
Anschließend wurden 0,20 % Methylparaben, 1,90 % Talkum, 0,90 % Farbpigmente bestehend aus 2 Teilen Tudor Mahagony, 4 Teilen Tudor Aspen, 3 Teile Tudor Rosewood, 6 Teile Tudor Willow , erhältlich z.B. bei der Fa. Croda Nettetal; sowie 4,00 % plättchenförmiger Pigmente mit vom Betrachtungswinkel abhängiger Farbigkeit zugemischt und alles homogen vermischt.

Die plättchenförmigen Pigmente mit vom Betrachtungswinkel abhängiger Farbigkeit wurden hergestellt, wie in Beispiel 3 von EP-A-686 674 beschrieben, mit dem Unterschied, daß die Schichtdicke 5 µm statt 7 µm beträgt und die eingesetzte Pigmentfraktion durch vierminütige Mahlung in einer Universalmühle und anschließendes Sieben mit einem Analysensieb mit einer Maschenweite von 50 µm gewonnen wurde.

Anschließend können noch nach Belieben Duftstoffe in einer Menge von 0,1 bis 1 % zugefügt werden (erhältlich z.B. bei der Fa. Orissa Drebing, Hamburg).

Der Gesichtspuder, bewirkt auf der Haut einen leicht glitzernden Bronzeeffekt mit einer ausgeprägten von rot nach grün umschlagenden Farbschattierung und ein sehr gutes Hautfeeling.

### Beispiel 2: Mascara

### Teil A)

5,00 % eines stearylgruppenhaltigen, wachsartigen Siloxans, erhältlich unter der Bezeichnung Wacker-Belsil SM 6018 bei der Fa. Wacker-Chemie, München
4,00 % eines phenylgruppenhaltigen Polydimethylsiloxanes, erhältlich unter der Bezeichnung Wacker-Belsil PDM 200 bei der Fa. Wacker-Chemie, München
5,00 % Cetylalkohol
7,00 % Stearinsäure
3,50 % Vaseline
4,50 % Paraffinöl dickfl.

### Teil B)

0,90 % Triethanolamin
65,10 % Wasser

### Teil C)

3,00 % Pigment-Farbstoff, z.B Tudor Ebony von der Fa. BASF

### Teil D)

2,00 % plättchenförmiger Pigmente mit vom Betrachtungswinkel abhängiger Farbigkeit (Korndurchmesser 10 bis 70 µm)

Die plättchenförmiger Pigmente mit vom Betrachtungswinkel abhängiger Farbigkeit wurden hergestellt wie in Beispiel 4 von EP-A-686 674 beschrieben, mit dem Unterschied, daß die Schichtdicke 5 µm statt 7 µm beträgt und die eingesetzte Pigmentfraktion durch vierminütige Mahlung in einer Universalmühle und anschließendes Sieben mit einem Analysensieb mit einer Maschenweite von 66 µm gewonnen wurde.

Teil A wurde bei 60°C aufgeschmolzen, Teil B unter schnellem Rühren eingemischt, anschließend wurde Teil C homogen eingearbeitet, anschließend Teil D eingemischt.

Die Mascara war cremig, schwarz mit grünlichem Schimmer mit einem Farbumschlag von grün nach blau.

### Beispiel 3: Mascara

Die Herstellung der Mascara erfolgte wie in Beispiel 2 beschrieben mit dem Unterschied, daß andere plättchenförmiger Pigmente mit vom Betrachtungswinkel abhängiger Farbigkeit eingesetzt wurden und in einer Konzentration von 3% statt 2%.

Die hier verwendeten plättchenförmiger Pigmente mit vom Betrachtungswinkel abhängiger Farbigkeit wurden hergestellt wie in Beispiel 3 von EP-A-686 674 beschrieben, mit dem Unterschied, daß die Schichtdicke 5 µm statt 7 µm beträgt und die eingesetzte Pigmentfraktion durch vierminütige Mahlung in einer Universalmühle und anschließendes Sieben mit einem Analysensieb mit einer Maschenweite von 66 µm gewonnen wurde.

Die Mascara war cremig, schwarz mit rotgoldenem Schimmer mit einem Farbumschlag nach grün, der stärker ausgeprägt war als der in Beispiel 2 beschriebene Farbumschlag.

### Beispiel 4: Lippenstift

2,00 % Bienenwachs
6,00 % Carnaubawachs
5,50 % Candelillawachs
6,50 % Ozokerite
1,50 % mikrokristallines Wachs
5,50 % Paraffinöl dickfl.
3,00 % Vaseline
5,00 % eines stearylgruppenhaltigen, wachsartigen Siloxans, erhältlich unter der Bezeichnung Wacker-Belsil SM 6018 bei der Fa. Wacker-Chemie, München
15,00 % Lanolinöl, erhältlich von der Fa. Grünau unter der Bezeichnung Fluilan
35,00 % Ricinusöl
5,00 % Überfettungsmittel z.B. erhältlich von der Fa. Goldschmidt unter der Bezeichnung Tegosoft 189
3,00 % eines phenylgruppenhaltigen Polydimethylsiloxanes, erhältlich unter der Bezeichnung Wacker-Belsil PDM 1000 bei der Fa. Wacker-Chemie, München
4,00 % Farbpigment, erhältlich von der Fa. BASF unter der Bezeichnung Sicomet-Rot 30
3,00 % plättchenförmiger Pigmente mit vom Betrachtungswinkel abhängiger Farbigkeit (Korndurchmesser 8 - 30 µm)
Die hier verwendeten plättchenförmigen Pigmente mit vom Betrachtungswinkel abhängiger Farbigkeit wurden hergestellt wie in Beispiel 3 von EP-A-686 674 beschrieben, mit dem Unterschied, daß die Schichtdicke 5 µm statt 7 µm beträgt und die eingesetzte Pigmentfraktion durch siebenminütige Mahlung in einer Universalmühle und anschließendes Sieben mit einem Analysensieb mit einer Maschenweite von 25 µm gewonnen wurde.

Alle Komponenten wurden vermischt und gemeinsam aufgeschmolzen.

Der Lippenstift glitzert leicht und liefert auf der Haut dezente Farbeffekte mit einem Farbumschlag von rot nach gold.

### Beispiel 5: Lippenstift

Die Herstellung des Lippenstifts erfolgte wie in Beispiel 4 beschrieben mit dem Unterschied, daß als Farbpigment Sicomet Red 15850 Ca erhältlich von der Fa. BASF eingesetzt wurde und daß andere plättchenförmige Pigmente mit vom Betrachtungswinkel abhängiger Farbigkeit eingesetzt wurden.

Die hier verwendeten plättchenförmigen Pigmente mit vom Betrachtungswinkel abhängiger Farbigkeit wurden hergestellt, wie in Beispiel 4 von EP-A-686 674 beschrieben, mit dem Unterschied, daß die Schichtdicke 5 µm statt 7 µm beträgt und die eingesetzte Pigmentfraktion durch siebenminütige Mahlung in einer Universalmühle und anschließendes Sieben mit einem Analysensieb mit einer Maschenweite von 25 µm gewonnen wurde.

Der Lippenstift schimmert grünlich und liefert auf der Haut dezente Farbeffekte mit einem Farbumschlag von orange nach lila.

### Beispiel 6: Haargel

### Teil A)

1,20 % Acrylamid/Acrylat Copolymer, erhältlich bei der Fa. Hoechst unter der Bezeichnung Hostacerin PN 73
1,20 % Polyvinylpyrrolidon, erhältlich z.B. bei der Fa. BASF unter der Bezeichnung Luviskol VA 64

### Teil B)

20,00 % Isopropanol
2,00 % phenylgruppenhaltiges Polydimethylsiloxan, erhältlich unter der Bezeichnung Wacker-Belsil PDM 20 bei der Fa. Wacker-Chemie, München
71,60 % Wasser

### Teil C)

4,00 % plättchenförmiger Pigmente mit vom Betrachtungswinkel abhängiger Farbigkeit (Korndurchmesser 30 - 110 µm)

Die hier verwendeten plättchenförmigen Pigmente mit vom Betrachtungswinkel abhängiger Farbigkeit wurden hergestellt, wie in Beispiel 4 von EP-A-686 674 beschrieben, wobei die Schichtdicke 5 µm statt 7 µm beträgt und die eingesetzte Pigmentfraktion durch dreiminütige Mahlung in einer Universalmühle und anschließendes Sieben mit einem Analysensieb mit einer Maschenweite von 100 µm und Siebung der erhaltenen Siebfraktion mit einem zweiten Analysensieb mit einer Maschenweite von 32 µm als Überstand gewonnen wurde.

Teil B wurde gemischt, Teil A langsam in Teil B eingemischt, und anschließend Teil C zugemischt.

Das Haargel schimmert im Glas und auf der Haut grünlich und sorgt in schwarzem Haar für starke Effekte bei einem Farbumschlag von grün nach blau, in hellem Haar sind die Effekte etwas dezenter.

### Beispiel 7: Self-foaming Lotion

### Teil A)

81,50 % Wasser
1,00 % Acrylsäurepolymer, erhältlich z.B. von der Fa. B.F. Goodrich unter der Bezeichnung Carbopol 934
2,50 % Vinylacetat/Ethylen Copolymer, unter der Bezeichnung Vinnapas RE 526-Z bei der Fa. Wacker-Chemie, München

### Teil B)

1,00 % Triethanolamin

### Teil C)

2,50 % Natriumlaurylsulfat, erhältlich z.B. unter der Bezeichnung Texapon N 40 von der Fa. Henkel, Düsseldorf
9,00 % niederviskoses, flüchtiges Polydimethylsiloxan, unter der Bezeichnung Wacker-Belsil DM 0.65 erhältlich bei der Fa. Wacker-Chemie, München
0,50 % glucosefunktionelles Polydimethylsiloxan, erhältlich unter der Bezeichnung SPG 121 VP bei der Fa. Wacker-Chemie, München
2,00 % plättchenförmige Pigmente mit vom Betrachtungswinkel abhängiger Farbigkeit (Korndurchmesser 20 bis 260 µm).

Die hier verwendeten plättchenförmigen Pigmente mit vom Betrachtungswinkel abhängiger Farbigkeit wurden hergestellt wie in Beispiel 3 von EP-A-686 674 beschrieben, mit dem Unterschied, daß die Schichtdicke 5 µm statt 7 µm beträgt und die hier eingesetzte Pigmentfraktion durch zweiminütige Mahlung in einer Universalmühle und anschließendes Sieben mit einem Analysensieb mit einer Maschenweite von 250 µm gewonnen wurde.

Teil A wurde gut vermischt bis eine homogene Lösung entstanden war, anschließend wurde mit Teil B neutralisiert und Teil C eingemischt.

Die self-foaming Lotion ist stark glitzernd und zeigt auf der Haut einen besonders stark ausgeprägten Rot-Grün-Farbumschlagseffekt.

### Beispiel 8: Sonnenschutzlotion

### Teil A)

6,00 % Emulgatormischung, käuflich erhältlich z.B. unter der Bezeichnung Teginacid von der Fa. Goldschmidt
1,00 % Isopropylmyristat
1,00 % Polydimethylsiloxan, erhältlich unter der Bezeichnung Wacker-Belsil DM 350 bei der Fa. Wacker-Chemie, München
4,00 % Paraffinöl dünnfl.
1,00 % Cetylstearylalkohol, erhältlich z.B. unter der Bezeichnung Lanette O von der Fa. Henkel, Düsseldorf

### Teil B)

10,00 % Kombination eines hochviskosen Polydimethylsilicongummis mit
   leichtflüchtigen cyclischen Polydimethylsiloxanen, erhältlich unter der Bezeichnung Wacker-Belsil CM 1000 bei der Fa. Wacker-Chemie, München
2,00 % UV-Absorber, z.B. erhältlich bei der Fa. Givaudan-Roure unter der Bezeichnung Parsol MCX

### Teil C)

71,50 % Wasser
1,50 % Glycerin

### Teil D)

2,00 % plättchenförmige Pigmente mit vom Betrachtungswinkel abhängiger Farbigkeit (Korngröße 10 - 55 µm).

Die hier verwendeten plättchenförmigen Pigmente mit vom Betrachtungswinkel abhängiger Farbigkeit wurden hergestellt wie in Beispiel 4 B von EP-A-686 674 beschrieben, mit dem Unterschied, daß die hier eingesetzte Pigmentfraktion durch fünfminütige Mahlung in einer Universalmühle und anschließendem Sieben mit einem Analysensieb mit einer Maschenweite von 50 µm gewonnen wurde.

Teil A und Teil C wurden je auf 65 - 70°C erhitzt. Teil C wurde in Teil A eingerührt, anschließend Teil B bei ca. 40°C eingemischt. Teil D wurde in die fertige Formulierung homogen eingearbeitet.

Die Sonnenschutzlotion zeigt dezente Farbeffekte auf der Haut mit einem Farbumschlag von grün nach blau.

### Beispiel 9: Sonnenschutz-Stift

### Teil A)

67,00 % Vaseline
25,00 % stearoxyfunktionelles wachsartiges Polydimethylsiloxan, erhältlich unter der Bezeichnung Wacker-Belsil SDM 6022 bei der Fa. Wacker-Chemie, München

### Teil B)

4,00 % UV-Absorber, erhältlich z.B. unter der Bezeichnung Parsol MCX von der Fa. Givaudan-Roure
4,00 % plättchenförmige Pigmente mit vom Betrachtungswinkel abhängiger Farbigkeit (Korndurchmesser 10 bis 55 µm).

Die hier verwendeten plättchenförmigen Pigmente mit vom Betrachtungswinkel abhängiger Farbigkeit wurden hergestellt wie in Beispiel 3 von EP-A-686 674 beschrieben, mit dem Unterschied, daß die Schichtdicke 5 µm statt 7 µm beträgt und die eingesetzte Pigmentfraktion durch fünfminütige Mahlung in einer Universalmühle und anschließendes Sieben mit einem Analysensieb mit einer Maschenweite von 50 µm gewonnen wurde.

Teil A wurde auf ca. 60°C erhitzt und gut vermischt, Teil B bei ca. 40°C eingemischt. Anschließend wurde die Mischung in die Form abgefüllt.

Der Sonnenschutzstift zeigt attraktive Bronzeeffekte auf der Haut mit einem Farbumschlag von rot nach grün. Im Bereich der UV-C- (200-230nm) und UV-B-Strahlung (290 - 330 nm) zeigte sich eine erhöhte Absorption im Vergleich zur Formulierung ohne plättchenförmiger Pigmente mit vom Betrachtungswinkel abhängiger Farbigkeit.

### Beispiel 10: Sonnenschutzstift

### Teil A)

62,00 % Vaseline
30,00 % stearoxyfunktionelles wachsartiges Polydimethylsiloxan, erhältlich unter der Bezeichnung Wacker-Belsil SDM 6022 bei der Fa. Wacker-Chemie, München

### Teil B)

4,00 % UV-Absorber, erhältlich z.B. unter der Bezeichnung Parsol MCX von der Fa. Givaudan-Roure
4,00 % plättchenförmige Pigmente mit vom Betrachtungswinkel abhängiger Farbigkeit (Korndurchmesser 10 bis 50 µm)

Die hier verwendeten plättchenförmigen Pigmente mit vom Betrachtungswinkel abhängiger Farbigkeit wurden hergestellt wie in Beispiel 4 von EP-A-686 674 beschrieben, mit dem Unterschied, daß die Schichtdicke 5 µm statt 7 µm beträgt und die eingesetzte Pigmentfraktion durch fünfminütige Mahlung in einer Universalmühle und anschließendem Sieben mit einem Analysensieb mit einer Maschenweite von 50 µm gewonnen wurde.

Teil A wurde auf ca. 60°C erhitzt, gut vermischt und abgekühlt. Bei ca. 40°C wurde Teil B eingemischt. Anschließend wurde die Mischung in die Form abgefüllt.

Der Sonnenschutz Stift ist ein weicher Stift, leicht grünlich schimmernd in der Verpackung und auf der Haut mit einem Farbumschlag nach blau. Er ist ideal als Lippenpflegestift mit Sonnenschutz.

### Beispiel 11: Farbloser Nagellack

### Teil A)

18,00 % Nitrocellulose, käuflich erhältlich z.B. bei der Fa. ICI, Frankfurt
4,00 % Phenylgruppenhaltiges Silsesquioxan, erhältlich bei der Fa. Wacker-Chemie, München unter der Bezeichnung Intermediate SY 430,
11,00 % Xylol
5,00 % Methylethylketon
22,00 % Ethylacetat
22,00 % Butylacetat

### Teil B)

2,00 % Campher
3,00 % Glykolsäurebutylester
1,00 % Diisopropyladipat, käuflich erhältlich z.B. bei der Fa. Dragoco, Holzminden unter der Bezeichnung Isoadipat
8,00 % Ethanol
2,00 % Phthalsäuredibutylester

### Teil C)

2,00 % plättchenförmige Pigmente mit vom Betrachtungswinkel abhängiger Farbigkeit (Korndurchmesser 100 bis 260 nm).

Die hier verwendeten plättchenförmigen Pigmente mit vom Betrachtungswinkel abhängiger Farbigkeit wurden hergestellt wie in Beispiel 4 von EP-A-686 674 beschrieben, mit dem Unterschied, daß die Schichtdicke 5 µm statt 7 µm beträgt und die hier eingesetzte Pigmentfraktion durch zweiminütige Mahlung in einer Universalmühle und anschließendem Sieben mit einem Analysensieb mit einer Maschenweite von 250 µm und anschließende zweite Siebung des Siebguts durch ein Analysensieb einer Maschenweite von 100 µm als Überstand gewonnen wurde.

Teil A wurde gut vermischt und gerührt bis eine homogene Mischung entstanden war. Teil B wurde homogen eingemischt und anschließend Teil C zugeben.

Der farblose Nagellack liefert besonders schöne Farbeffekte mit einem Farbumschlag von grün nach blau, der bei Auftragung über einem dunklen Unterlack noch verstärkt wird.

### Beispiel 12: Lidschatten-Kompaktpuder

### Teil A)

20,00 % Talkum
25,00 % Kaolin
5,00 % Titandioxid
10,00 % Calciumcarbonat
5,00 % Magnesiumstearat
5,00 % Bornitrid, erhältlich unter der Bezeichnung Wacker Bornitrid BNP bei der Fa. Wacker-Chemie, München
12,50 % Zinkstearat

### Teil B)

3,50 % Isopropylmyristat
4,00 % Oleyloleat, erhältlich z.B. unter der Bezeichnung Cetiol bei der Fa. Henkel, Düsseldorf

### Teil C)

5,00 % plättchenförmige Pigmente mit vom Betrachtungswinkel abhängiger Farbigkeit (Korndurchmesser 5 - 35 µm)

Die hier verwendeten plättchenförmigen Pigmente mit vom Betrachtungswinkel abhängiger Farbigkeit wurden hergestellt wie in Beispiel 4 von EP-A-686 674 beschrieben, mit dem Unterschied, daß die Schichtdicke 5 µm statt 7 µm beträgt und die hier eingesetzte Pigmentfraktion durch siebenminütige Mahlung in einer Universalmühle und anschließendes Sieben mit einem Analysensieb mit einer Maschenweite von 32 µm gewonnen wurde.
5,00 % Pigment-Farbstoff, erhältlich z.B. unter der Bezeichnung Cloisonne Blue 650 Z von der Fa. Mearl

Teil A gut vermischen, Teil B erhitzen und portionsweise einmischen, Teil C einmischen und gut homogenisieren, anschließend zu kleinen Blöcken verpressen.

Das Lidschatten-Kompaktpuder ist ein lilafarbenes Puder mit leicht grünlichem Schimmer und einem Farbumschlag nach blau.

### Beispiel 13: Duschgel

22,50 % Alkylamidopropylbetain, erhältlich z.B. bei der Fa. Hoechst unter der Bezeichnung Genagen CAB
53,50 % Wasser
22,50 % Ammoniumlaurylethersulfat, erhältlich z.B. bei der Fa. Henkel, Düsseldorf unter der Bezeichnung Texapon NA
1,00 % polyethermodifiziertes Polydimethylsiloxan, erhältlich bei der Fa. Wacker-Chemie unter der Bezeichnung Wacker-Belsil DMC 6038
0,50 % Ammoniumchlorid
2,00 % plättchenförmige Pigmente mit vom Betrachtungswinkel abhängiger Farbigkeit (Korndurchmesser 50 bis 260 µm)

Die hier verwendeten plättchenförmigen Pigmente mit vom Betrachtungswinkel abhängiger Farbigkeit wurden hergestellt wie in Beispiel 4 von EP-A-686 674 beschrieben, mit dem Unterschied, daß die Schichtdicke 5 µm statt 7 µm beträgt und die hier eingesetzte Pigmentfraktion durch dreiminütige Mahlung in einer Universalmühle und anschließendem Sieben mit einem Analysensieb mit einer Maschenweite von 250 µm und anschließende Siebung des Siebguts mit einem Analysensieb mit einer Maschenweite von 50 µm als Überstand gewonnen wurde.

Alle Komponenten wurden in der angegebenen Reihenfolge vermischt.

Das Duschgel ist markant grünlich schimmernd und zeigt einen starken Farbumschlag nach blau.

### Beispiel 14: Duschgel

Die Herstellung des Duschgels erfolgte wie in Beispiel 13 beschrieben mit dem Unterschied, daß 0,2 % plättchenförmiger Pigmente mit vom Betrachtungswinkel abhängiger Farbigkeit eingesetzt wurden.

Das Duschgel war im Vergleich zu dem Duschgel aus Beispiel 13 weniger stark grünlich schimmernd und zeigte einen stark dezenten Farbumschlag nach blau.

### Beispiel 15: Body Gel

### Teil A)

0,40 % eines Acrylsäurepolymers, käuflich erhältlich z.B. von der Fa. B.F.Goodrich unter der Bezeichnung Carbopol 94, 64,70 % Wasser.

### Teil B)

0,80 % eines polyethermodifizierten Polydimethylsiloxans, käuflich erhältlich bei der Fa. Wacker-Chemie, München unter der Bezeichnung Wacker-Belsil DMC 6031

### Teil C)

33,00 % Ethanol

### Teil D)

0,30 % Triethanolamin

### Teil E)

0,05 % Pigmente mit vom Betrachtungswinkel abhängiger Farbigkeit wie in Beispiel 10 verwendet.

Teil A wurde gut vermischt und auf 75°C erhitzt, Teil B wurde eingemischt und gut verrührt. Nach Abkühlen auf ca. 45°C wurde Teil C und anschließend Teil D eingemischt.

Das Body Gel war grünlich-blau schimmernd. Es ist hervorragend geeignet als Gel-Grundlage für medizinische Gels.

### Beispiel 16: Salbengrundlage

### Teil A)

9,00 % Cetylstearylalkohol, z.B. erhältlich bei der Fa. Henkel, Düsseldorf unter der Bezeichnung Lanette O
10,50 % Paraffinöl dickflüssig
10,50 % Vaseline, weiß
1,00 % Polydimethylsiloxan mit der Viskosität 350 mm²/s, erhältlich von der Fa. Wacker-Chemie, München unter der Bezeichnung Pharsil 350

### Teil B)

68,00 % Wasser

### Teil C)

1,00 % Pigmente mit vom Betrachtungswinkel abhängiger Farbigkeit wie in Beispiel 10 verwendet.

Teil A wurde auf ca. 65 - 70°C erhitzt. Teil B wurde erhitzt und in Teil A eingemischt und gut homogenisiert. Anschließend wurde Teil C homogen eingemischt.

Die erhaltene Salbengrundlage war grünlich-blau schimmernd und diente als cremige Grundlage zum Einarbeiten von pharmazeutischen Wirkstoffen mit ansprechendem Erscheinungsbild und hervorragendem Hautgefühl.

### Beispiel 17: Zahnpaste

19,00 % Wasser wurden vorgelegt und 0,50 % Natriumcarboxymethylcellulose, erhältlich z.B. bei der Fa. Hoechst unter der Bezeichnung Tylose CB 200, sowie 5,70 % hochdisperse Kieselsäure, erhältlich unter der Bezeichnung HDK N 20 P bei der Fa. Wacker-Chemie, München, unter Rühren zugeben.
4,30 % Polyethylenglykol 400, 17,00 % Sorbitollösung 70 %ig und 50,00 % Glycerin wurden eingerührt. 4,00 % Pigment mit vom Betrachtungswinkel abhängiger Farbigkeit wie in Beispiel 11 verwendet, 2,50 % Natriumlaurylsulfat, erhältlich z.B. unter der Bezeichnung Texapon K 1296 bei der Fa. Henkel, Düsseldorf wurden vorsichtig zugemischt. Dabei wurde starke Schaumbildung vermieden. Die fertige Formulierung wurde kurze Zeit evakuiert.

Es wurde eine transparente Zahnpaste mit interessantem Farbspiel von grün nach blau erhalten.

## Patentansprüche

1. Kosmetische oder pharmazeutische Zubereitungen enthaltend Pigmente mit vom Betrachtungswinkel abhängiger Farbigkeit und die für kosmetischen oder pharmazeutischen Zubereitungen üblichen Zusatzstoffe, dadurch gekennzeichnet, daß die Pigmente mindestens eine orientierte vernetzte Substanz mit flüssigkristalliner Struktur mit chiraler Phase enthalten, von plättchenförmiger Gestalt sind und eine Dicke von 1 - 20 µm haben.

2. Zubereitungen gemäß Anspruch 1, dadurch gekennzeichnet, daß die Pigmente eine Dicke von 4 - 8 µm haben.

3. Zubereitungen gemäß Anspruch 1, dadurch gekennzeichnet, daß die Pigmente eine Dicke von 4 - 6 µm haben.

4. Zubereitungen gemäß Anspruch 1, 2 oder 3, dadurch gekennzeichnet, daß die Pigmente einen Korndurchmesser von 5 - 500 µm haben.

5. Zubereitungen gemäß einem oder mehreren der Ansprüche 1 bis 3, dadurch gekennzeichnet, daß die Pigmente einen Korndurchmesser von 10 - 100 µm haben.

6. Zubereitungen gemäß einem oder mehreren der Ansprüche 1 bis 5, dadurch gekennzeichnet, daß die Pigmente in den erfindungsgemäßen Zusammensetzungen in Mengen von 0,05 - 40 % (w/w) vorliegen.

7. Zubereitungen gemäß einem oder mehreren der Ansprüche 1 bis 5, dadurch gekennzeichnet, daß die Pigmente in den erfindungsgemäßen Zusammensetzungen in Mengen von 0,1 - 20 % (w/w) vorliegen.

8. Zubereitungen gemäß einem oder mehreren der Ansprüche 1 bis 7, dadurch gekennzeichnet, daß die Pigmente aus Polyorganosiloxanen bestehen.

9. Zubereitungen gemäß einem oder mehreren der Ansprüche 1 bis 8, dadurch gekennzeichnet, daß Interferenzfarbpigmenten oder Metallglanzpigmenten zusätzlich in der Zubereitung vorhanden sind.

10. Zubereitungen gemäß einem oder mehreren der Ansprüche 1 bis 9, dadurch gekennzeichet, daß als Zusatzstoffe eine oder mehrere Substanzen ausgewählt aus der Gruppe tierische, mineralische, pflanzliche oder synthetische Öle, Wachse oder Harze, Netzmittel, Fettalkohole, Emulgatoren, Sonnenfilter, organische Lösungsmittel, Verdickungsmittel, Farbstoffe, Pigmente, pH-Regler, Reduktionsmittel, Elektrolyte, Trübungszusätze, Konservierungsmittel, Antioxidantien, Permuttiermittel, Parfums, antiseborrhoische Mittel und Wirkstoffe, die der Behandlung, der Pflege und dem Schutz der Haut oder der Haare dienen können, sowie Wasser vorhanden sind.
